# EUROPEAN PATENT SPECIFICATION

(11) **EP 2 704 119 B1**
(45) Date of publication and mention of the grant of the patent: **23.06.2021**
(21) Application number: 13182249.6
(22) Date of filing: 29.08.2013
(51) Int. Cl.: G08B 21/04, G08B 29/18

(54) **Fall detection**
Falldetektion
Détection de chute

(30) Priority: 29.08.2012 ES 201231342
(43) Date of publication of application: 05.03.2014
(73) Proprietor: Vodafone IP Licensing Limited, The Connection Newbury Berkshire RG14 2FN (GB); Vodafone España, S.A.U., 28042 Madrid (ES)
(72) Inventor: Rubio Andres, Francisco Javier, 28150 Madrid (ES); Alonso Diaz, Patricia, 28150 Madrid (ES); Almodovar Herraiz, Daniel, 28150 Madrid (ES); Esteve Asensio, Guillermo Bruno, 28150 Madrid (ES)
(74) Representative: Boult Wade Tennant LLP

(56) References cited:
- US-A1- 2009 315 719
- US-A1- 2009 322 540
- US-A1- 2012 083 237
- None

## Description

### BACKGROUND TO THE INVENTION

Fall detection using electronic devices is already known in the art. These are particularly widespread in the geriatric field, where it is often the case that a subject may fall and subsequently be unable to call for help. For example, US patent US7450332 discloses an integrated free-fall detection device for a portable apparatus where an acceleration sensor generates acceleration signals correlated to the components of the acceleration of the portable apparatus along three detection axes, said device encompassing a dedicated purely hardware circuit connected to the acceleration sensor, which generates a free-fall detection signal in a continuous manner and in real-time. The free-fall detection signal has a first logic value in the event that the acceleration signals are simultaneously lower than a respective acceleration threshold, and this value is sent to a processor unit of the portable apparatus as an interrupt signal to activate the appropriate actions of protection for the portable apparatus.

In another example, European patent EP1870037 discloses a portable apparatus for detecting falls and immobility of a subject, comprising at least one sensor that generates a signal indicative of the accelerations of at least one portion of the subject's body, a control unit suitable for processing this signal and communication means for sending a call for help to a remote control station. The control unit may automatically activate the communication means only when a fall of the subject and his/her subsequent immobility are detected based on said processing. Typically in fall detection apparatus, as exemplified by EP1870037, a guard time timer is provided that ensures that the apparatus is immobile after a sudden change in movement. In this way, false positives that are created purely as a result of sharp or exaggerated movement are reduced.

Present fall detectors implementations are typically based on specific devices such as those described above to be fixed to the wrist or the hip, which include an accelerometer and run certain mathematical algorithms in order to determine whether a fall event has occurred.

Such portable electronic devices which are designed to be worn near the hip on a belt or a clip on the belt are considered to be the most reliable ones because the hip is generally less prone to sporadic movement than a subject's limbs. Nevertheless, they generally have to rely on an indication that the device is locked onto the hip and otherwise not to activate the fall detection algorithm. Others such portable electronic devices that are designed to be worn on the wrist, for example, on watches, generally provide a lower detection success rate and is accountable for more false positive results than devices designed to be worn near the hip because the device is not so close to the body's center of gravity and the movement is generally less stable.

US 2009/315,719 A1 relates to a fall accident detection apparatus and method capable of detecting frequent falling accidents in daily lives of old, feeble or invalid pedestrians in real time and notifying a fall control server of the falling accidents in order to actively prepare for possible secondary accidents by falls.

US 2009/322,540 A1 relates to a system, a method and an apparatus for autonomous monitoring, detecting and tracking of movement and orientation of a body or portion of a body.

A problem with the current techniques for detecting a fall event is that the percentage of successful fall detections is not adequately high and the percentage of false positive detections is not adequately low. A false positive detection may be understood as a positive detection event that has been triggered incorrectly, i.e. a positive output is given although a fall event has not actually occurred. It is therefore an object of the present invention to provide a method, system and device for detecting fall event with improved accuracy, offering high rate of successful fall detections and a low rate of false positive detections.

### SUMMARY OF THE INVENTION

According to an aspect of the present disclosure, there is provided a method in a mobile electronic device for handling fall detection, the method comprising: detecting a suspected fall; and, preventing or suspending, at least temporarily, a triggering of a fall process based on a status of being used and/or historical status of being used of the mobile electronic device, the status of being used and/or historical status of being used being at the time the suspected fall was detected. In this way, false positives are reduced as the device may prevent or suspend the process that occurs once a fall has been detected. If it is unlikely that a fall occurred, but rather a drop or similar, then the fall that has been detected can be ignored. The present disclosure is operable to utilise conventional fall detection algorithms.

The status of being used and/or historical status of being used of the mobile electronic device may comprise: an input event being detected during a predetermined time period prior to the suspected fall being detected. The input event may be actuation of a button on the mobile electronic device. Alternatively, the input event may be detection of a touch on a touch screen of the mobile electronic device. In this way, if the device was being used prior to the fall, then a drop is more likely to have occurred rather than a fall have occurred when the user is walking along, i.e. not using the device.

The status of being used and/or historical status of being used of the mobile electronic device may comprise: a voice call being ongoing. The triggering of the fall process may be suspended until termination of the voice call. The triggering of the fall process may be prevented if the voice call is maintained for a predetermined amount of time after the detection of the suspected fall. If a call is ongoing, an alert should not be sent generating a false positive. After call hangup, the user may be presented with an alert to confirm if an alert should not be sent.

The status of being used and/or historical status of being used of the mobile electronic device may comprise: a screen lock process occurring during a predetermined time period prior to the suspected fall being detected.

The method may further comprise: detecting movement of the mobile electronic device; determining, based on the detected movement, whether or not a user of the mobile electronic was walking at the time or prior to, during a predetermined time period, the suspected fall being detected; and, preventing the triggering of the fall process where the user was not walking. Thus the fall alert is prevented when a fall is unlikely to have occurred and has been generated accidentally such as a drop of the device or similar. Only when the user is walking is a fall likely to have occurred and therefore only in those circumstances should an alert be generated. As above, the present disclosure dramatically reduces the chances of false positives being generated.

The fall process may comprise: initialising a guard time timer for confirming that a fall has occurred; upon expiry of the guard time timer, informing the user that a fall has been detected and initialising a cancellation time timer; and, before expiry of the cancellation time timer, providing the user with an opportunity to prevent an alert from being sent to a security system.

A corresponding mobile electronic device and computer readable medium are also provided.

### BRIEF DESCRIPTION OF THE DRAWINGS

Preferred embodiments of the present invention will now be described with reference to the accompanying drawings, in which:
Figure 1 shows a mobile fall detection method;
Figure 2 shows an example implementation of the algorithm of the present invention;
Figure 3 shows an exemplary flow chart of the of the present invention; and
Figure 4 shows another example flow chart of an embodiment of the method of the present invention.

### DETAILED DESCRIPTION

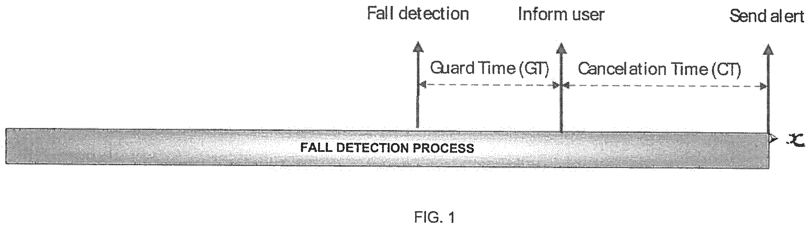

Referring to Figure 1, an example overview of a known mobile fall detection method is shown. Time is shown along the x-axis.

The device may be fixed to the wrist, held at the hip, held in the hand, or otherwise held any other conceivable position of the subject's body. The device comprises means for detecting a fall event, for example, using at least one accelerometer. The device may be a specific device that is dedicated to detecting fall events, or it may be a non-specific device, for example, a mobile phone or a tablet device, that has the capability of detecting fall events. Preferably, the device provides sufficient sensitivity and an adequate refresh rate in order to detect fall events.

In the example a positive fall detection result is triggered, for example, based on one or several abrupt change(s) in acceleration. A guard time (GT) timer is initiated during which the device detects whether there is any further movement of the subject. Alternatively, the GT timer may be initiated when the device detects that the subject is lying on the ground, for example, by detecting the relation position (x,y,z) of the subject to the ground. At the end of the GT, the subject may be informed that a positive fall detection result is currently set.

Considering that sudden changes in acceleration and/or detection that a subject being close to the ground may not necessarily indicate that a fall event has actually occurred, a cancelation time (CT) timer may also be initiated, which gives the subject a certain amount of time to cancel a positive fall detection result. The CT timer may start subsequent to the end of the GT timer (as shown in Figure 1), or it may be initiated at the same time as the GT timer (not shown) and comprise a longer time period than the GT. During the CT, the subject may voluntarily and manually cancel the positive fall detection result (before or after the device informs the subject of such a result), for example if he believes that no fall event has occurred or if he does not wish for the device to send a signal for help.

At the end of the CT, the device may automatically send a signal for help if the subject has not cancelled this function, for example, for the reasons described above. In order to send a signal or alert to, for example, a remote device, the device may be communicatively coupled to said remote device.

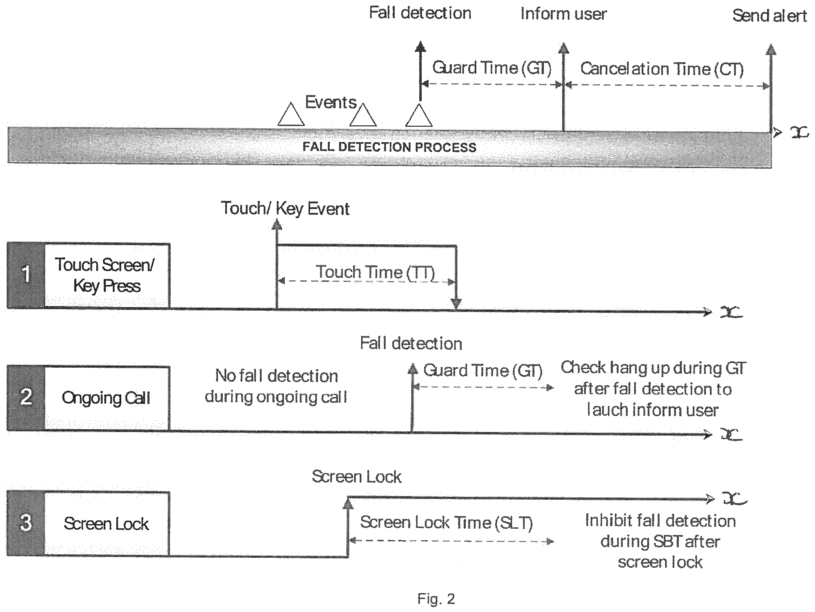

Referring now to Figure 2, example implementations of the algorithm of the present invention are shown. These may be implemented alone or, preferably, in addition, for example, in parallel or sequentially, to the method as shown in Figure 1. Time is shown along the x-axis.

The device on which the method of the present invention is performed (for example, the methods shown in Figure 2) is a non-specific mobile device. Implementations based on specific devices are dedicated only to fall detection and this could mean that the subject may forget to wear the device; the subject may not be expecting to fall constantly. Non-specific devices, for example, a mobile phone or tablet, may be more likely to be carried by the subject on a routine basis. Previous attempts to move towards non-specific devices with the capability of detecting fall events have generally failed because some usual movements done to use the non-specific device may result in a false positive fall detection result. However, the present invention offers algorithms, examples of which will be described below, that provide additional accuracy and reliability required to overcome this problem. Figure 2 shows a first example of an algorithm of the present invention. Say, for example, that the device is a mobile device that utilises a touch screen as a user interface. The algorithm of Figure 2 checks whether the touch screen or a key on the touch screen has been pressed. Once such a touch screen press event has been detected, a touch time (TT) timer is initiated, during which a fall detection event will not be triggered, i.e. the fall detected event is over-ridden.

Figure 2 shows a second example of an algorithm of the present invention. Say, for example, that the device is a mobile phone. The algorithm of Figure 2 checks for ongoing calls of the mobile phone. During ongoing calls, if the fall detection event is activated and a positive output results, the algorithm of Figure 2 will check at the end of the GT that the call is maintained. If yes, the positive fall detection result will be cancelled; if no, the user is informed that a positive fall detection result is currently set and the subject is given a CT in order to cancel the positive fall detection results in the same manner as described above for Figure 1. If, say at the end of the CT, the subject has not cancelled the positive fall detection result, then a signal or alert is sent to a remote device in the same manner as described above for Figure 1.

Figure 2 shows a third example of an algorithm of the present invention. Say, for example, that the device is a mobile phone that may be locked. The algorithm of Figure 2 checks for the event that the mobile phone is locked. If yes, a screen lock time (SLT) timer is initiated, and the fall detection means is over-ridden. The SLT timer ends when it is detected that the mobile phone has been unlocked. If no, fall detected occurs in the same manner as shown in Figure 1.

It will be appreciated that each of the methods of Figure 2 may be performed separately, in combination with each other, or in combination with any other algorithm of the present invention.

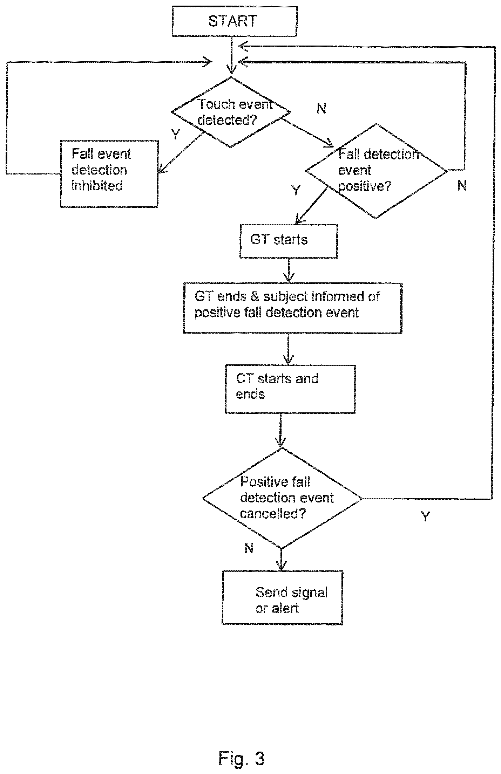

Figure 3 shows an example flow chart of the on-top algorithm of the present invention, corresponding to Figure 2.

A device and a method will now be described for detecting a fall of the user bearing the device. The fall detection is accomplished by determining a certain number of events and different responses to said events.

An object of the invention proposes a solution to fall detection devices offering a low rate of false positives. An object of the invention solves said problem by providing a mobile electronic device and a method of managing said device allowing the mobile electronic device to activate the fall detection event reducing the false positive percentage and at the same time allowing the use of a mobile device that you are used to charging and wearing every day like a mobile phone.

The quality of a fall detector method comes from the percentage of successful fall detections and the percentage of false positives. The first one should be high: around 90% is a good value for the different fall types. The second should be minimum: values of 5% are acceptable for the daily activities. The current state of the art shows that nobody has provided these values using mobile phones acting as the electronic device used to determine a fall event, since users perform movements with the mobile phone that lead to reduce the fall detection success or increase the percentage of false positives.

This document proposes both a mobile device and a method, which actually works on top of any given fall detection procedure furnished at any similar device, based in specific mobile phone assets to allow the use of a mobile phone as a fall detector device with the same reliability than specific devices as those mentioned in the art worn on the hip.

One of the most important requirements is that of the mobile Z electronic device comprising acceleration detection means, namely accelerometer. Accelerometers of mobile phones are considered sensitive enough, and have a suitable refresh rate, to be used by any standard fall detector procedure. Since the fall detector method and device hereby described are mainly based in abrupt changes in the acceleration and a guard time (GT) where no movement is detected in the body, they may be also base the alarm in the relative position (x,y,z) of the device related to the floor to detect a person lying down.

Bearing in mind that falls or sudden changes in acceleration or relative position may happen, the object of the invention, both the device itself and the method, may account an additional amount of time given to user to voluntarily and manually cancel the fall detection alert cancellation time (CT).

In order to avoid false positives the solution hereby posed takes into account the amount of acceleration changes detected, the calibration provided and the sensitivity grader tuned by the parameters,), reducing the possibility of false positives. As earlier stated the method of the invention works on top of any other fall detection method or in combination with the latter.

Preferably, other processes based on the accelerometer itself, may be checked or monitored, for example checking that the person is walking to activate the fall detection event with security. Finally the use of the accelerometer as acceleration detection means and any others contexts or parameters potentially monitored by the hardware of the device might be used to provide an automatic variation of the fall detector parameters or even the modification of the fall detector method depending on the user context.

Apart from the accelerometer, there are others mobile assets that might be used as context indicator related to different triggers or timers, for example:
- GPS that could be used for indoor or outdoor detection, and attaching geo-referenced information to the fall alarm once the event is triggered.
- Launch a text-to-speech message to ask about the user health status and record the response in order to process it and detect specifics words or silence, thus triggering the event i.e. the accelerometer detects a sudden change in acceleration a TTS is launched, no answer is received a timer is set a TTS Timer, after the time allocated to said time and no response identified or checked as positive, the fall alarm is then triggered.
- Power charging. To avoid more false positives, the processing unit might check and monitor the battery level of the device.
- Camera. The processing unit might command a built-in camera activate and with a single algorithm decide what to do if the captured image is, for example, a dark or the sky or a face. The same principal could also apply to a video, for security and to provide more information the camera can start to record video and audio while the text-to-speech is trying to capture information around.
- Any combination of the above.

A preferred embodiment of the invention is depicted by the Figures where the fall detection method of the invention is running, in said picture the skilled person may account for several processes, some are running in parallel whilst others are running sequentially even in a conditional way.

In this preferred embodiment a fall detection event occurs when a fall is detected by an accelerometer, said event is acknowledged (thus an alarm signal is sent) or discarded depending on the results of checking several processes or subprocesses being carried on by a mobile fall detection electronic device, in this case a mobile phone equipped with:
- wireless communication means operative to create at least a connection to a radio communication network for establishing at least a voice call,
- at least one acceleration detection module,
- at least one timer operative to manage timebased events on the device by means of a processing unit,
- and input/output means operative to allow the input or output of data for this preferred embodiment a touch screen and at least one key/button are selected.

The processing unit of the device is connected to the at least one timer and can actuate to suspend, at least temporarily, a fall detection trigger, thus a sending of alert, for a certain period of time after at least one specific context (touch time, guard time, screen lock time, cancellation and time cancellation request, or a status and/or historical status of the device itself) is determined by the at least one timer (touch time timer, guard time timer, screen lock time timer, cancellation time timer). Hence the processing unit starts a process to check and monitor whether the touch screen is being actuated or any key pressed in the mobile, once that the screen is touched and during the timer called touch time (TT) the fall detection activation event is discarded. If no input is detected on the screen acting input/output means then the processing unit performs a process to monitor and check ongoing calls on the mobile phone. During ongoing calls if the fall detector event is activated, the process will check during the timer called guard time (GT) a period of time that the ongoing call is maintained and, after that, it will discard or not the fall detector event depending on the screen lock time timer (SLT); after the screen lock process is launched the screen lock time timer and until expire all the fall detection events will be discarded.

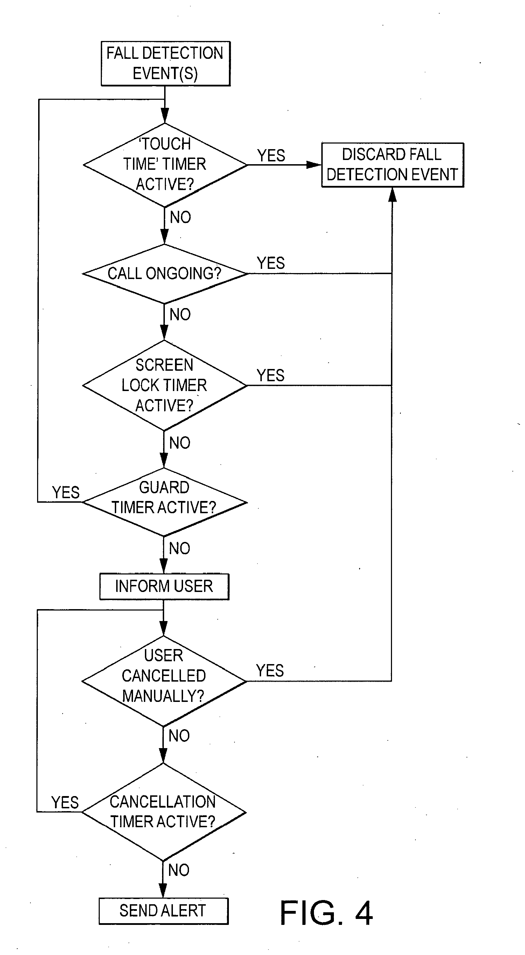

Referring now to Figure 4, another example of a flow chart of an embodiment of the method of the present invention is shown.

In this flow chart the fall detection method is ready for sending an alert using a mobile electronic devices with wireless communication capabilities, the method as seen in said Figure 4 the method does not send any alert before suspending at least temporarily a fall detection trigger, thus the sending of the alert depends [at least for a certain period of time] on after at least one specific context to be determined; and the result of said context allows the determination of another context or the assumption that no fall event is detected.

Since the method establishes priorities to the context and events, the response to a certain context condition the determination of the next, that is to say contexts are dependent and sometimes the response to one of the excludes the next to be determined.

Referring to Figure 4, the skilled person may notice that the first specific context to be determined is related to a touch time timer associated to input means of the mobile electronic device; the determination of said context set the fall detection event as discarded when the touch time timer is determined to be activated whereas if it is determined to be deactivated a second context is monitored. When the touch time timer is determined to be deactivated, the processing unit of the mobile electronic device (mobile phone) determines whether an ongoing call is established on said mobile electronic device or not, if an on going call is determined to be established on the mobile phone then the fall detection event is discarded, if not the processing unit follows the method depicted by Figure 4 and starts determining whether a screen lock time timer is activated or not. If the screen lock time timer is determined to be deactivated then the fall detection method starts over again from the beginning, determining the touch time timer, if not the processing unit proceed with informing the user of the triggering of the fall detection event by means of the input/output means...a beep, a message on the screen, a blink of a led, or any visible or audible message.

Once the user is properly informed he or she may trigger a cancellation request in response to the information received, i.e. it's been a false positive since the user was just playing on the ground, then the fall detection event if discarded. If the user does not perform the cancellation then the processing unit invokes the next context to check whether a cancellation time timer is activated or not; if the cancellation time timer is determined to be activated the processing unit will trigger the cancellation request again, if not an alert will be sent.

In other example of the present invention (not shown in the Figures), other processes based on the accelerometer itself, may be checked or monitored, for example, checking that the subject is walking before activating the fall detection event. For example, a fall event detection that follows from a subject who is determined to be walking at the time may be more likely to be a real fall event as opposed to a situation where the subject is determined to be still at the time that the fall event is detected.

## Claims

1. A method in a mobile electronic device for handling fall detection, the method comprising:
detecting a suspected fall; and,
preventing or suspending, at least temporarily, a triggering of a fall process based on a status of being used and/or historical status of being used of the mobile electronic device, the status of being used and/or historical status of being used being at the time the suspected fall was detected.

2. A method according to any preceding claim, wherein the status of being used and/or historical status of being used of the mobile electronic device comprises:
an input event being detected during a predetermined time period prior to the suspected fall being detected.

3. A method according to claim 2, wherein the input event is actuation of a button on the mobile electronic device.

4. A method according to claim 2, wherein the input event is detection of a touch on a touch screen of the mobile electronic device.

5. A method according to any preceding claim, wherein the status of being used and/or historical status of being used of the mobile electronic device comprises:
a voice call being ongoing.

6. A method according to claim 5, wherein the triggering of the fall process is suspended until termination of the voice call.

7. A method according to claim 6, wherein the triggering of the fall process is prevented if the voice call is maintained for a predetermined amount of time after the detection of the suspected fall.

8. A method according to any preceding claim, wherein the status of being used and/or historical status of being used of the mobile electronic device comprises a screen lock process occurring during a predetermined time period (SLT) prior to the suspected fall being detected.

9. A method according to any preceding claim, further comprising:
detecting movement of the mobile electronic device;
determining, based on the detected movement, whether or not a user of the mobile electronic device was walking at the time or prior to, during a predetermined time period, the suspected fall being detected; and,
preventing the triggering of the fall process where the user was not walking.

10. A method according to any preceding claim, wherein the fall process comprises:
initialising a guard time (GT) timer for confirming that a fall has occurred;
upon expiry of the guard time (GT) timer, informing the user that a fall has been detected and initialising a cancellation time (CT) timer; and,
before expiry of the cancellation time (CT) timer, providing the user with an opportunity to prevent an alert from being sent to a security system.

11. A mobile electronic device comprising means for carrying out the method of any of claims 1 to 10.

12. A computer readable medium containing instructions which when executed by a processor of a mobile electronic device cause the mobile electronic device to carry out the method of any of claims 1 to 10.

## Patentansprüche

1. Ein Verfahren in einer mobilen elektronischen Vorrichtung zur Handhabung der Fallerkennung, wobei das Verfahren Folgendes umfasst:
Erkennen eines vermuteten Falls; und
Verhindern oder Aussetzen, zumindest vorübergehend, einer Auslösung eines Prozesses bei Fallvorgängen basierend auf einem Status der Verwendung und/oder einem historischen Status der Verwendung der mobilen elektronischen Vorrichtung, wobei der Status der Verwendung und/oder der historische Status der Verwendung zur gleichen Zeit auftreten, zu der der vermutete Fall erkannt wurde.

2. Ein Verfahren nach einem vorhergehenden Anspruch, wobei der Status der Verwendung und/oder der historische Status der Verwendung der mobilen elektronischen Vorrichtung Folgendes umfassen:
ein Eingabeereignis, das während eines zuvor bestimmten Zeitraums vor der Erkennung des vermuteten Falls erkannt wird.

3. Ein Verfahren nach Anspruch 2, wobei es sich bei dem Eingabeereignis um die Betätigung einer Taste auf der mobilen elektronischen Vorrichtung handelt.

4. Ein Verfahren nach Anspruch 2, wobei es sich bei dem Eingabeereignis um die Erkennung einer Berührung auf einem Berührungsbildschirm der mobilen elektronischen Vorrichtung handelt.

5. Ein Verfahren nach einem vorhergehenden Anspruch, wobei der Status der Verwendung und/oder der historische Status der Verwendung der mobilen elektronischen Vorrichtung Folgendes umfassen:
einen laufenden Sprachanruf.

6. Ein Verfahren nach Anspruch 5, wobei die Auslösung des Prozesses bei Fallvorgängen ausgesetzt wird, bis der Sprachanruf beendet ist.

7. Ein Verfahren nach Anspruch 6, wobei die Auslösung des Prozesses bei Fallvorgängen verhindert wird, wenn der Sprachanruf für eine zuvor bestimmte Zeitdauer nach der Erkennung des vermuteten Falls aufrechterhalten wird.

8. Ein Verfahren nach einem vorhergehenden Anspruch, wobei der Status der Verwendung und/oder der historische Status der Verwendung der mobilen elektronischen Vorrichtung Folgendes umfassen:
einen Bildschirmsperrvorgang, der während eines zuvor bestimmten Zeitraums (SLT) vor der Erkennung des vermuteten Falls erfolgt.

9. Ein Verfahren nach einem vorhergehenden Anspruch, weiter umfassend:
Erkennen der Bewegung der mobilen elektronischen Vorrichtung;
Bestimmen, basierend auf der erkannten Bewegung, ob ein Benutzer der mobilen elektronischen Vorrichtung zum Zeitpunkt der oder vor der Erkennung des vermuteten Falls während eines zuvor bestimmten Zeitraums gegangen ist; und
Verhindern der Auslösung des Prozesses bei Fallvorgängen, wenn der Benutzer nicht gegangen ist.

10. Ein Verfahren nach einem vorhergehenden Anspruch, wobei der Prozess bei Fallvorgängen Folgendes umfasst:
Initialisieren eines Überwachungszeittimers (Guard Time, GT-Timer) zur Bestätigung, dass ein Fall stattgefunden hat;
nach Ablauf des Überwachungszeittimers (Guard Time, GT-Timer), Benachrichtigen des Benutzers, dass ein Fall erkannt wurde, und Initialisieren eines Aufhebungszeittimers (Cancellation Time, CT-Timer); und
vor Ablauf des Aufhebungszeittimers (Cancellation Time, CT-Timer), Ermöglichen, für den Benutzer, das Senden einer Warnung an ein Sicherheitssystem zu verhindern.

11. Eine mobile elektronische Vorrichtung umfassend Mittel zum Ausführen des Verfahrens nach einem der Ansprüche 1 bis 10.

12. Ein computerlesbares Medium beinhaltend Anweisungen, die bei Ausführung durch eine Verarbeitungseinheit einer mobilen elektronischen Vorrichtung veranlassen, dass die mobile elektronische Vorrichtung das Verfahren nach einem der Ansprüche 1 bis 10 durchführt.

## Revendications

1. Un procédé dans un dispositif électronique mobile destiné à la gestion d'une détection de chute, le procédé comprenant :
la détection d'une chute suspectée, et
l'évitement ou la suspension, au moins temporairement, d'un déclenchement d'un processus de chute en fonction d'un état d'utilisation et/ou d'un état historique d'utilisation du dispositif électronique mobile, l'état d'utilisation et/ou l'état historique d'utilisation étant à l'instant où la chute suspectée a été détectée.

2. Un procédé selon l'une quelconque des Revendications précédentes, où l'état d'utilisation et/ou l'état historique d'utilisation du dispositif électronique mobile comprend :
un événement d'entrée qui est détecté au cours d'une période temporelle prédéterminée antérieure à la détection de la chute suspectée.

3. Un procédé selon la Revendication 2, où l'événement d'entrée est l'actionnement d'un bouton sur le dispositif électronique mobile.

4. Un procédé selon la Revendication 2, où l'événement d'entrée est la détection d'une pression sur un écran tactile du dispositif électronique mobile.

5. Un procédé selon l'une quelconque des Revendications précédentes, où l'état d'utilisation et/ou l'état historique d'utilisation du dispositif électronique mobile comprend :
un appel vocal qui est en cours.

6. Un procédé selon la Revendication 5, où le déclenchement du processus de chute est suspendu jusqu'à la terminaison de l'appel vocal.

7. Un procédé selon la Revendication 6, où le déclenchement du processus de chute est évité si l'appel vocal est maintenu pendant une durée prédéterminée après la détection de la chute suspectée.

8. Un procédé selon l'une quelconque des Revendications précédentes, où l'état d'utilisation et/ou l'état historique d'utilisation du dispositif électronique mobile comprend
un processus de verrouillage d'écran se déroulant au cours d'une période temporelle prédéterminée (SLT) antérieure à la détection de la chute suspectée.

9. Un procédé selon l'une quelconque des Revendications précédentes, comprenant en outre :
la détection d'un déplacement du dispositif électronique mobile,
la détermination, en fonction du déplacement détecté, si ou non un utilisateur du dispositif électronique mobile marchait à l'instant de ou antérieurement à, au cours d'une période temporelle prédéterminée, la détection de la chute suspectée, et
l'évitement du déclenchement du processus de chute si l'utilisateur ne marchait pas.

10. Un procédé selon l'une quelconque des Revendications précédentes, où le processus de chute comprend :
l'initialisation d'une horloge de temps de garde (GT) destinée à la confirmation qu'une chute s'est produite,
à l'expiration de l'horloge de temps de garde (GT), l'information de l'utilisateur qu'une chute a été détectée et l'initialisation d'une horloge de temps d'annulation (CT), et
avant l'expiration de l'horloge de temps d'annulation (CT), la fourniture à l'utilisateur d'une opportunité d'éviter l'envoi d'une alerte à un système de sécurité.

11. Un dispositif électronique mobile comprenant un moyen d'exécution du procédé selon l'une quelconque des Revendications 1 à 10.

12. Un support lisible par ordinateur contenant des instructions qui, lorsqu'elles sont exécutées par un processeur d'un dispositif électronique mobile, amènent le dispositif électronique mobile à exécuter le procédé selon l'une quelconque des Revendications 1 à 10.
